# EUROPEAN PATENT APPLICATION

(11) **EP 1 522 853 A1**
(43) Date of publication of application: **13.04.2005**
(21) Application number: 03733543.7
(22) Date of filing: 23.06.2003
(51) Int. Cl.: G01N 33/53, G01N 37/00, G01N 27/62

(54) **DNA MICROARRAY HAVING STANDARD PROBE AND KIT CONTAINING THE ARRAY**

(30) Priority: 24.06.2002 JP 2002183249; 28.06.2002 JP 2002191390
(71) Applicant: CANON KABUSHIKI KAISHA, Ohta-ku Tokyo 146-8501 (JP)
(72) Inventor: KAWAGUCHI, Masahiro, c/o CANON KABUSHIKI KAISHA, Tokyo 146-8501 (JP); OKAMOTO, Tadashi, c/o CANON KABUSHIKI KAISHA, Tokyo 146-8501 (JP); TAKASE, Hiromitsu, c/o CANON KABUSHIKI KAISHA, Tokyo 146-8501 (JP); HASHIMOTO, Hiroyuki, c/o CANON KABUSHIKI KAISHA, Tokyo 146-8501 (JP)
(74) Representative: Beresford, Keith Denis Lewis
(86) International application number: PCT/JP2003/007918
(87) International publication number: WO 2004/001412

(57) **Abstract**

To provide a DNA micro-array having a nucleic acid probe immobilized on a substrate, which is used to detect a molecule of a target nucleic acid contained in a sample and has a substantially complementary base sequence to the target base sequence of the nucleic acid molecule, including at least one probe selected from the group consisting of: at least one internal standard probe for assay of PCR of the target nucleic acid; at least one external standard probe for a detection operation and assay of an amount of the probe; and a probe for measurement of the amount or density of the nucleic acid probe, formed by the same method as the nucleic acid probe.

SEQUENCE LISTING

<110> Canon Kabushiki Kaisha

<120> DNA microarray provided with reference probe and array kit

<130> 2942530

<160> 18

<170> PatentIn version 3.1

<210> 1
<211> 18
<212> DNA
<213> Artificial

<220>
<223> probe

<400> 1
actggccgtc gttttaca 18

<210> 2
<211> 20
<212> DNA
<213> Artificial

<220>
<223> probe

<400> 2
gtgagctatg agaaagcgcc 20

<210> 3
<211> 20
<212> DNA
<213> Artificial

<220>
<223> probe

<400> 3
ggtatcttta tagtcctgtc 20

<210> 4
<211> 20
<212> DNA
<213> Artificial

<220>
<223> probe

<400> 4
ggccttttgc tcacatgttc 20

<210> 5
<211> 18
<212> DNA
<213> Artificial

<220>
<223> external standard

<400> 5
tgtaaaacga cggccagt 18

<210> 6
<211> 20
<212> DNA
<213> Artificial

<220>
<223> external standard

<400> 6
ggcgctttct catagctcac 20

<210> 7
<211> 20
<212> DNA
<213> Artificial

<220>
<223> external standard

<400> 7
gacaggacta taaagatacc 20

<210> 8
<211> 20
<212> DNA
<213> Artificial

<220>
<223> external standard

<400> 8
gaacatgtga gcaaaaggcc 20

<210> 9
<211> 40
<212> DNA
<213> Artificial

<220>
<223> probe

<400> 9
tttttttttt tttttttttt tttttttttt tttttttttt 40

<210> 10
<211> 18
<212> DNA
<213> Artificial

<220>
<223> primer

<400> 10
gagacaataa ccctgata 18

<210> 11
<211> 18

<212>  DNA
<213>  Artificial

<220>
<223>  primer

<400>  11
ccttaacgtg agttttcg                                                    18


<210>  12
<211>  18
<212>  DNA
<213>  Artificial

<220>
<223>  primer

<400>  12
gcttggagcg aacgacct                                                    18


<210>  13
<211>  18
<212>  DNA
<213>  Artificial

<220>
<223>  primer

<400>  13
gagtcgacct gcaggcat                                                    18


<210>  14
<211>  20
<212>  DNA
<213>  Artificial

<220>
<223>  primer

<400>  14
ggttggactc aagacgatag                                                  20


<210>  15
<211>  18
<212>  DNA
<213>  Artificial

<220>
<223>  primer

<400>  15
taagttgggt aacgccag                                                    18


<210>  16
<211>  18
<212>  DNA
<213>  Artificial

<220>
<223>  primer

<400>  16

agggcgctgg caagtgta                                                    18

<210>  17
<211>  18
<212>  DNA
<213>  Artificial

<220>
<223>  primer

<400>  17
cgtttcggtg atgacggt                                                    18

<210>  18
<211>  20
<212>  DNA
<213>  Artificial

<220>
<223>  primer

<400>  18
gcggtaatac ggttatccac                                                  20

## Description

### TECHNICAL FIELD

The present invention relates to a DNA micro-array useful for quantitative analysis of a target nucleic acid (target nucleic acid hereinafter) in a sample. More specifically, the present invention relates to a DNA micro-array that can improve accuracy in a quantitative analysis using a DNA micro-array of nucleic acid probes having a sequence expected to be complementary to a target nucleic acid sequence in a sample. The present invention also relates to a detection kit using the DNA micro-array.

### BACKGROUND ART

As a result of development in methods for detecting and measuring genetic DNA on the basis of hybridization process, rapid progress has been made in studies of gene chip (DNA chip, micro-array)where a plurality of nucleic acid probes are immobilized on a substrate, allowing multiple detection tests on genetic DNA in a sample concomitantly. The method of detecting and measuring genetic DNA contained in a sample using a gene chip (DNA chip, micro-array) is expected to be applied in various fields such as molecular biological researches, and gene disorder and infectious disease diagnoses.

The basic form of a gene chip is that a plurality of single-stranded nucleic acid fragments expected to have a complementary base sequence to the base sequence of a target gene are arranged in an array and immobilized on the surface of a substrate such as a glass substrate in order to detect target genetic DNA by the hybridization process. Single-stranded nucleic acid fragment to be used as a probe having a complementary base sequence to a target gene can be a chemically synthesized DNA oligomer called "oligo-DNA" or a complementary strand DNA fragment called "cDNA" enzymatically synthesized using a gene derived from a biomaterial as a template. As for immobilization of oligo-DNA on the surface of the substrate, methods are roughly classified into two: one is, as shown in US 5,474,796 B (or JP 9-500568 A, ProtoGene Laboratories), to synthesize DNA molecules on the substrate sequentially after immobilizing one end to the substrate, the other is, as disclosed in JP H11-187900 A (Canon), oligo-DNA are synthesized, and then various bonding means are used to immobilize these nucleic acid fragments on a substrate. As described above, the immobilizing method is roughly classified into above two methods.

As the means for immobilizing the separately prepared nucleic acid fragments on the substrate, various methods have been proposed, such as an adsorption immobilizing method making use of the charge of a substrate and the charge of a nucleic acid fragment, and an immobilizing method which is aimed to improve immobilization efficiency by using a coating film formed by coating the substrate surface with a poly-L-lysine or amino silane coupling agent.

Currently, DNA micro-arrays are not provided with special probes for quantitative analysis of nucleic acid having a target sequence (target nucleic acid). Whether the DNA micro-array is a DNA micro-array formed on the surface of a solid phase by a sequential synthesis method or a DNA micro-array produced by a pin method, there are variations caused by the production process, for example, variation in synthesis efficiency due to the nucleic acid sequence, variation in the amount of the probe liquid dropped from a pin spotter on a substrate or variation in immobilization rate. Therefore, there are problems in quantification accuracy and reproducibility. An ink jet production method has been proposed (JP H11-187900 A) to solve the variation at the time of DNA micro-array production.

On the other hand, a sample to be examined by the DNA micro-array is subjected to an amplification operation to amplify a target nucleic acid from the viewpoint of sensitivity, as well as to incorporate a detectable label to into the amplified product. The basic technology of this amplifying operation is called "PCR" and disclosed in US 4683195 B, US 4683202 B and US 4965188 B. However, PCR involves hard-to-control factors in the amplification reaction process. For example, it is well known that even when the same samples are amplified at the same time, the amplification yield may differ by several times to several tens of times. Studies have been made to solve this uncertainty in the amplification yield, providing competitive PCR etc. The competitive PCR is detailed in reports by P.D. Siebert et al. (Nature 359; 557-558 (1992), Bio Techniques 14; 244-249 (1993)). As an alternative method, a known amount of a certain nucleic acid is added as an internal standard to a sample taken from a patient, and the internal standard nucleic acid and the target nucleic acid are amplified in order to estimate the amplification rate of the target nucleic acid from the amplification rate of the internal standard nucleic acid.

### DISCLOSURE OF THE INVENTION

As described above, when a quantitative analysis of a target nucleic acid is carried out using a DNA micro-array, there exist various unstable factors that affect the determination.

First of all, there is low reliability of accuracy and reproducibility due to variation in the amount of a probe immobilized on a substrate during production of a DNA micro-array. There is also variation in the amplification rate of a target nucleic acid in a sample due to the uncertainty of the amplification efficiency of the target nucleic acid in PCR. Conventionally, variations in these different factors are calibrated by various methods separately, and then quantitative analysis is carried out using a DNA micro-array, and the analytical data are corrected on the basis of the calibration results for quantitative determination.

Since these calibration procedures are very complicated and time-consuming, they are inhibiting factors in processing a large number of samples.

The present invention is to solve the above problems. It is therefore an object of the present invention to provide a DNA micro-array which allows obtainment of correction data at the time of quantitative determination of the amount of a target nucleic acid in a sample, greatly simplifying the calibration procedures, as well as an assay kit used for the above purpose.

The inventors of the present invention have studied intensively to solve the above problems and have found that these assay works can be greatly simplified if a DNA micro-array is prepared by applying and immobilizing separately prepared nucleic acid probes using an ink-jet method (JP H11-187900 A) and at least either a probe for an internal standard nucleic acid (internal standard probe hereinafter) or a probe for an external standard nucleic acid (external standard probe hereinafter) is immobilized together with the nucleic acid probes for detecting target nucleic acid. Based on the above findings, the inventors of the present invention have also devised a kit for carrying out these assay works using the above DNA micro-array accurately and easily to complete the present invention.

Thus, according to the present invention, there is provided A DNA micro-array for detecting nucleic acid molecules having target base sequences in a sample comprising a substrate and nucleic acid probes having base sequences substantially complementary to the target base sequences immobilized on the substrate, wherein the array contains additional probes of one or several kinds selected from the following probes:
probes for internal standard nucleic acids (internal standard probes) of one or several kinds which hybridize with said internal standards and are added for quantitative evaluation of PCR of said nucleic acid molecules having the target base sequences; probes for external standard nucleic acids (external standard probes) of one or several kinds which hybridize with said external standards and are added for evaluation of accuracy of detection operation and for quantitative analysis of the amount of the probes having base sequences substantially complementary to the target base sequences;
and probes added for quantitative evaluation of the amount or density of said nucleic acid probes having base sequences substantially complementary to the target base sequences, which are immobilized by the same method as said nucleic acid probes.

Preferably, probes for internal standard nucleic acids and probes for external standard nucleic acids is immobilized on the substrate at four probe concentrations or densities to form a group.

Preferably, the internal standard probe to be immobilized on the DNA micro-array comprises at least two kinds of probes corresponding to amplification products of two different chain lengths obtained from the internal standard nucleic acid. Also the external standard probe comprises preferably at least two kinds of probes of an appropriate base number being complementary to the external standard nucleic acid to be added.

More preferably, the internal standard probe and the external standard probe are synthetic nucleic acids immobilized on the substrate. The chain length of the synthetic nucleic acid is 15 to 75 bases preferably.

Further, when the DNA micro-array according to the present invention contains an internal standard probe, an internal standard nucleic acid is amplified together with the target nucleic acid in one amplification reaction to determine the amplification efficiency of the reaction. The present invention also provides a primer set for an amplification reaction of an internal standard nucleic acid. That is, the primer set of the present invention is for amplification of an internal standard nucleic acid to be amplified together with a target nucleic acid and is designed to generate an amplified product having a chain length similar to the amplification product of the target nucleic acid.

The present invention also provides a detection kit suitable for quantitative detection of a target nucleic acid using a DNA micro-array of the present invention, which includes a primer set for the amplification reaction of the internal standard nucleic acid to be amplified together with the target nucleic acid. When the amplification product of the target nucleic acid is of two or more chain lengths, the kit includes at least two primer sets as claimed in claim 7, corresponding to the chain lengths. The primer sets include at least one primer set to obtain an amplification product of 200 bp or less in chain length, at least one primer set for an amplified products of 200 to 500 bp, at least one primer set for 500 to a 2,000 bp amplification product, and at least one primer set for a 2,000 bp or more amplification product.

Preferably, according to the present invention, a kit for detecting a target base sequence includes internal standard nucleic acid to be amplified together with a target nucleic acid in an amplification reaction for quantitative detection of a target nucleic acid using the DNA micro-array, where the internal standard nucleic acid is two or more nucleic acids derived from a microorganism or a virus having no homology with the target base sequence to be detected.

The present invention also provides a detection kit containing external standard nucleic acid, suitably used for the quantitative detection of a target nucleic acid using the DNA micro-array of the present invention containing external standard probes. That is, according to the present invention, there is provided a kit for detecting a target base sequence, including at least two external standard nucleic acids to be added to a sample upon quantitatively detecting a target nucleic acid using a DNA micro-array, where the external standard nucleic acids are synthetic nucleic acids labeled with a detectable marker. The marker is preferably a fluorescent material, a radioactive material or a luminescent material.

In the DNA micro-array of the present invention, the nucleic acid probe includes a single-stranded nucleic acid, specifically DNA, RNA, PNA (peptide nucleic acid), cDNA (complementary DNA) or cRNA (complementary RNA). Further, the DNA micro-array may comprise a single-stranded nucleic acid as a nucleic acid probe and a target nucleic acid hybridized to the nucleic acid probe.

### BRIEF DESCRIPTION OF THE DRAWINGS

Fig. 1 shows an internal standard probe, external standard probe, external standard nucleic acid and internal standard nucleic acid, a primer set for amplification of an internal standard nucleic acid and amplified products thereof according to an embodiment of the present invention;
Fig. 2 schematically shows an arrangement of internal standard probes in the DNA micro-array according to an embodiment of the present invention;
Fig. 3 is a graph showing calibration curves for calculating the probe immobilization rate using external standard probes in the DNA micro-array in quantitative analysis using the DNA micro-array according to the present invention;
Fig. 4 is a graph showing calibration curves for calculating the PCR amplification factor using internal standard probes provided in the DNA micro-array in quantitative analysis using the DNA micro-array according to the present invention, the graph also showing a difference in detection sensitivity according to the chain length of the nucleic acid of the amplified product;
Fig. 5 schematically shows the plane arrangement and sectional form (taken along the line A-A) of a biochip as a structural example according to the present invention; and
Fig. 6 is a graph showing the relationship between the measured secondary ion intensity and DNA formation density (number of DNA molecules per probe dot) of a biochip.

### BEST MODE FOR CARRYING OUT THE INVENTION

According to a first aspect of the present invention, there is provided a DNA micro-array having an internal standard probe formed thereon.

In a broader sense, the internal standard probe is a probe for detecting an internal standard nucleic acid to be used to assist quantitative determination of a target nucleic acid. This prove is a nucleic acid probe having a base sequence complementary to an internal standard nucleic acid or gene of which amount in a sample is known and can bind to it, to enable quantitative determination of a target nucleic acid on the basis of the relative ratio of the internal standard nucleic acid to the target nucleic acid bound to the array. For example, a transcription product of a gene encoding a cytoskeleton (a house keeping gene) is often used as the internal standard nucleic acid because its expression amount hardly changes and is relatively large. Specific examples of the transcription product include mRNA of GAPDH or of β-actin and ribosomal RNA.

In a narrower sense, the internal standard is a nucleic acid of a known base sequence to be added in a sample in a known amount when an amplification reaction of nucleic acid (e.g., PCR) is carried to increase the amount of nucleic acid or to label the target nucleic acid with a detectable marker. Such an internal standard nucleic acid to be added to the sample is selected so as to be amplified or labeled at the same efficiency as the target nucleic acid when amplification or labeling of the target nucleic acid is carried out. After the amplification or labeling reaction, the amount of the internal standard nucleic acid is determined to calculate the amplification factor or labeling rate so that the amount of the target nucleic acid can be obtained as a relative value based on the amount of the internal standard nucleic acid.

According to a second aspect of the present invention, there is provided a DNA micro-array having a probe for an external standard nucleic acid (external standard probe) formed thereon.

Here, the external standard nucleic acid is a nucleic acid having a known base sequence to be added to a sample in order to compensate variation in the DNA micro-array production step and/or in the detection and measurement step. More specifically, it is a nucleic acid prepared by introducing a detectable and determinable marker into a highly purified nucleic acid such as synthesized DNA or plasmid DNA, of which labeling rate etc. are determined in advance. This external standard nucleic acid may be used for any type of DNA micro-arrays not depending to the type of target genes so long as it has no base sequence homology to the base sequence of the target nucleic acid. The external standard probe is a nucleic acid probe having a base sequence complementary to the external standard nucleic acid and can selectively bind to the external standard.

Plural sets of external standard nucleic acids and external standard probes may be used at the same time to determine the performance of the DNA micro-array, that is, quantification ability thereof. More specifically, a DNA micro-array having not less than three, preferably not less than five external standard probes is prepared, and labeled external standard nucleic acids corresponding to these probes are added in known but different amounts when the target nucleic acid is detected. Calibration curves can be prepared by plotting the detected and added amounts of these external standard nucleic acids. The amount of a probe immobilized on a DNA micro-array can also be corrected by using a set of external standard nucleic acid and external standard probe. That is, variation in probe immobilization efficiency between DNA micro-arrays (substrates) can be corrected using such a set. In this method, an external standard probe is immobilized on a DNA micro-array in not less than three concentrations, preferably not less than five concentrations. By reacting a sufficient amount of the labeled external standard nucleic acid with this array and determining the amounts of the bound external standard nucleic acid, the ratio of the probe molecules actually immobilized on the DNA micro-array can be determined.

According to a third aspect of the present invention, there is provided a detection kit that makes a detection and determination method utilizing the DNA micro-array of the first or second aspect more efficient and easy.

The detection kit that uses a DNA micro-array having an internal standard probe comprises a primer set for the amplification of an internal standard nucleic acid. The detection kit using a DNA micro-array having an external standard probe includes an external standard nucleic acid labeled with a detectable and determinable marker and whose labeling rate has been determined. According to the internal standard probes or external standard probes, at least one, optionally several to ten or more primer sets or external standard nucleic acids are included in the detection kit.

The content of an primer set to amplify an internal standard nucleic acid varies depending on various requirements. First of all, the base sequences (position) of the primer set must be determined to produce an amplified product having a complementary base sequence to the internal standard probe in an amplification reaction using an internal standard nucleic acid as a template. At the same time, care must be taken not to generate undesirable products by the amplification reaction using the target nucleic acid as a template. Further, in order to improve accuracy as an internal standard, it should be avoided that the chain length of the amplified product of the target nucleic acid greatly differs from that of the internal standard amplified with the primer set in this kit. More specifically, it is desired that the several amplified products differing in length are generated from the internal standard nucleic acid so that one of them is equal in length to the amplified product of the target nucleic acid. For example, four primer sets are prepared to obtain four amplified products of 200 bp or less, 200 to 500 bp, 500 to 2,000 bp, and 2000 bp or more, so as to select one having the same chain length as the amplified product of the target nucleic acid. Of course, more primer sets may be prepared more finely dividing the range of the chain length of the amplified products.

Preferable internal standard nucleic acid may be a nucleic acid of a known base sequence and high purity. Examples of such nucleic acid are commercially available nucleic acids for molecular biological and medical use, such as plasmid vectors, phage DNA and microbial genomes. In particular, plasmid vectors and phage DNA are preferred because they are available as a highly purified and homogeneous sample relatively easily.

On the other hand, the external standard nucleic acid labeled with a detectable and determinable marker with a known labeling rate may be also prepared using a nucleic acid having a known base sequence and of high purity such as synthetic DNA or a plasmid vector. Synthesized DNA is particularly preferred because of its stable labeling efficiency and high labeling rate per molecular weight.

Fruthermore, the DNA micro-array of the present invention preferably has preferably both the internal standard probe and the external standard probe. In this case, the detection kit preferably includes the external standard nucleic acid as well as a primer set for the amplification of the internal standard nucleic acid.

As long as the external standard probe and the internal standard probe are treated separately, the same probes (sequence) can be used for them. When they are used at the same time, probes of different base sequences (different species) must be used because competition occurs between them. Density standard probes may serve as the external standard probes.
[Analyzing method using a density standard probe formed by the same method as the nucleic acid probe, added for assaying the amount or density of the nucleic acid probe]

The present invention further provides a method of analyzing a DNA array itself. The following embodiment is given to specifically illustrate the present invention.

Conventional quantitative analysis, for example, the analysis using TOF-SIMS has a problem that detected secondary ion intensity differs according to measurement conditions, namely, primary ion application conditions (acceleration energy, incident angle, ion species, and application amount) and secondary ion detection conditions (energy width), and when a sample is an insulator, elimination by an electron beam pulse-applied for the correction of charge and the degree of charge neutralization.

In particular, when the sample is an insulator, charge conditions are not always the same between a standard sample and a sample to be measured when primary ions are applied to them. Therefore, to achieve a high level quantitativity, measurement conditions must be optimized precisely every time the sample is changed. When optimization is unsatisfactory, the accuracy of the measurement results is not obtained. Creation of a calibration curve requires standard samples as many as the concentration levels to be measured, so that it requires a troublesome work.

When standard samples for a nucleic acid chip are prepared by spin coating as reported by P. Lazzeri et al., high density reproducibility can be obtained if averaged in a wide area, but uniformity of the coating is not necessary satisfactory in view of the minute area size of TOF-SIMS measurement as small as several tens to several hundreds µm², which gives a serious problem to the reliability of measurement results. To solve the above problem, the present invention provides a method for highly quantitative analysis of a so-called nucleic acid chip where a plurality of nucleic acids (probes) are arranged in a matrix pattern on a substrate, by using time-of-flight type secondary ion mass spectrometry (TOF-SIMS). The present invention also provides a DNA micro array which allows easy determination of the amount or density of nucleic acid of each probe dot arranged on the nucleic acid chip, by using TOF-SIMS.

The inventors of the present invention have conducted intensive studies to solve the above problem. They have found that when nucleic acid probe dots of known average fixation density as a density standard and nucleic acid probe dots of unknown fixation density are formed by the same method on the surface of a substrate, and the density standard dots are subjected to the secondary ion mass spectrometry to detect secondary signals, it is possible to determine with highly reproducible quantitativity the nucleic acid concentration of probe dots of unknown concentration on a substrate by secondary ion mass spectrometry, on the basis of the signal intensity of the density standard dots.

Further, the DNA micro-array of this embodiment, can contain a probe of a single-stranded nucleic acid hybridized with a target nucleic acid on the substrate.

In addition, this embodiment provides a method of producing a DNA micro-array having nucleic acid probes having a plurality of nucleic acid-related substances arranged in a matrix pattern on a substrate, which comprises the step of forming nucleic acid probe dots of known average nucleic acid density per dot as density standards on part of the surface of a substrate on which the nucleic acid probe dots have been formed. In addition, this method comprises the steps of: forming nucleic acid probe dots in a matrix pattern on the substrate; and forming nucleic acid dots of known average nucleic acid density per dot as density standard on part of the surface of the substrate, on which the nucleic acid probe dots have been already formed, by the same method with the probe dots. The DNA micro-array of this embodiment is characterized in that it has an area where dots of nucleic acid of known concentrations are arranged in addition to the nucleic acid probes arranged in a matrix pattern on the substrate. A calibration curve is generated based on the secondary ionic intensity by TOF-SIMS from that area, and then the amount of the nucleic acid probes (formed density) of a probe dot in another area on the DNA micro-array substrate is determined from its secondary ion intensity by using the calibration curve.

That is, in the DNA micro-array of this embodiment, dots of nucleic acid probe of which per dot density is known are formed on part of a substrate on which a plurality of nucleic acid-related substances are arranged in a matrix pattern (so-called DNA micro-array), and they are used as the density standard for quantitative measurement by TOF-SIMS. A calibration curve can be drawn instantly if nucleic acid probe dots being step-wisely different in concentration are formed on the DNA micro-array substrate, enabling more quantitative measurement. In general, the nucleic acid density per dot of the nucleic acid probe dots of known density is separately determined by chemically analyzing probe dots prepared separately but in the same conditions.

The DNA micro-array of the present invention is generally produced by using a flat substrate of a size of 1 cm × 1 cm, 1 inch × 1 inch (25.4 mm × 25.4 mm) or of a slide glass size (for example, 26 mm × 76 mm) on which a matrix of probe dots are arranged. As described above, the present invention allows highly reliable TOF-SIMS determination of the density of the nucleic acid probe immobilized on respective dots arranged in a matrix pattern on the surface of a DNA micro-array, utilizing the above density standards formed on the same substrate.

In the DNA micro-array of this embodiment, nucleic acid probes arranged on the substrate are preferably immobilized on the substrate by covalent bonding as described later. For this purpose, it is effective to treat the surface of the substrate with (N-β-(aminoethyl)-γ-aminopropyltrimethoxysilane), a silane coupling agent having an amino group bonded thereto, and N-maleimidocaproyloxysuccinimido (EMCS), a crosslinking agent in order to form a coating film in the process of treating the surface of the substrate.

In chemical analysis for determining the amount of nucleic acid contained per dot, a plurality of DNA micro-arrays having a large number of nucleic acid probe dots are prepared, by changing the nucleic acid concentration stepwise, and then these micro-arrays of a certain concentration are treated with an acid, for example, to separate the nucleic acid probes from the substrate, and the total amount of the nucleic acid probes contained in this solution is determined separately by analyzing the trace amounts of phosphorus contained in the nucleic acid with microanalysis means. The microanalysis means used to determine the trace amounts of phosphorus contained in the nucleic acid is not particularly limited but preferably one having high sensitivity and high accuracy, such as Inductively Coupled Plasma Mass Spectrometry (ICP-MS). The detection limit of phosphorus by ICP-MS is about 10 ppb. For determination, the amount of phosphorus must be at least 2 to 3 times as much as the above amount, that is, about 20 to 30 ppb. The minimum amount of a sample (amount of the solution) is 1 ml.

When nucleic acid probes are formed by an ink jet method described later, it is not difficult to form dots of nucleic acid probes in a matrix of 200 × 300, for example, in an area of 1 inch × 3 inch. By the ink jet method, the amount of one discharge is highly reproducible and an average value thereof (order of pl) can be estimated with high reliability. The concentration of probe DNA (order of µM) in the discharged solution used for the production of the above density standard dots is predetermined and of course known.

The total number of the nucleic acid probe dots to be solubilized for the substrates to obtain the sample amount required for the above ICP-MS can be estimated to a certain extent by using, in addition to the above values, the immobilized amount of the probe DNA (the amount obtained by subtracting the amount of probe DNA unimmobilized due to washing with water etc. from the discharged amount, a level of several tens of percents). More specifically, when a nucleic acid chip is produced under the above typical conditions, several tens of DNA micro-arrays having a matrix size of about 200 rows × 300 columns and a discharge rate per dot in the order of pl must be used for each concentration determination (order of µM).

As for dots formed as density standards on the DNA micro-array, the dot number of one known concentration is not particularly limited but it is preferable to form plural dots.

In this embodiment, the nucleic acid density of the probe dots of unknown density can be determined by using a calibration curve prepared from the signal intensities of secondary ions obtained from the nucleic acid probe dots changing the density stepwise. In the analyzing method of the present invention, it is effective to use secondary ion intensity detected by the time-of-flight type secondary ion mass spectrometry for higher quantitativity.

The above secondary ion intensity is not a counting rate but preferably integrated intensity counted for a certain time under certain conditions. To be exact, the dose of the primary ion is preferably set to a certain value of 1 × 10¹²/cm² or less which is called "static conditions", and a count value of the following secondary ions released from a certain area is used as the secondary ion intensity. The dose of the primary ion must be not more than 1 × 10¹⁴/cm².

Examples of the primary ion used for measurement by TOF-SIMS include Cs⁺ ion, Ga⁺ ion and Ar⁺ ion. Examples of the secondary ion species include P⁻, PO⁻, PO₂⁻ and PO₃⁻ derived from phosphate constituting the skeleton of a nucleic acid when the probe nucleic acid and the target nucleic acid introduced by hybridization are single-stranded nucleic acids, specifically DNA, RNA, cDNA (complementary DNA) or cRNA (complementary RNA). Detectable secondary ions are bases from which a proton has been eliminated, i.e., C₅H₄N₅⁻ (134 a.m.u.) in case of adenine, in the case of guanine, C₅H₉N₅O⁻ (150 a.m.u.), in the case of cytosine, C₄H₄N₃O⁻ (110 a.m.u.) and in the case of thymine, C₅H₅N₂O₂⁻ (125 a.m.u.). When the nucleic acid probe is an RNA probe, the same secondary ion as the DNA probe can be detected except that C₄H₃N₂O₂⁻ (111 a.m.u.) derived from uracil can be detected in place of thymine. When an insulating substrate such as a glass substrate is used as the substrate for the DNA micro-array, suitable application conditions must be determined for electron beam irradiation used in combination with primary ion beam irradiation in consideration of the pulse width and frequency of the primary ion, the dielectric constant of the sample and the thickness of the glass substrate.

The analyzing method of the DNA micro-array of the present invention is also characterized in that the secondary ion intensity derived from a nucleic acid can be quantitatively displayed as a 2-D image in accordance with the scanning using the primary ion application.

### Examples

The following examples are provided for the purpose of further illustrating the present invention in more detail. These examples are just examples of the best embodiment according to the present invention but are in no way to be taken as limiting.

### Example 1

### <Preparation of internal standard nucleic acid>

A plasmid DNA of a known base sequence, pUC118 (3,162 bp, a product of Takara), was used as an internal standard nucleic acid. To be a PCR template, the above plasmid pUC118 was cleaved by EcoRI restriction enzyme, in accordance with a predetermined method to prepare linear DNA. After confirming the condition of digestion by using agarose gel electrophoresis, the reaction solution was treated with phenol, and the linear DNA of interest was collected by precipitation with ethanol. The collected linear DNA was dissolved in a TE buffer solution (10 mM Tris-HCl (pH of 7.5), 1 mM EDTA) to a final concentration of 10 ng/µl (2.4 pmol/ml) to prepare a solution of internal standard nucleic acid.

### Example 2

### <Preparation of primers for the amplification of internal standard nucleic acids>

As shown in Table 1 below, five forward primers P1 to P5 and four reverse primers RP1 to RP4 were selected as PCR primers on the basis of information on the base sequence of the linear pUC118 digested by EcoRI prepared in Example 1.

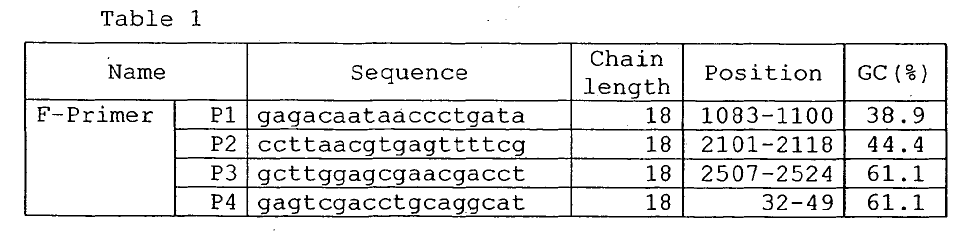

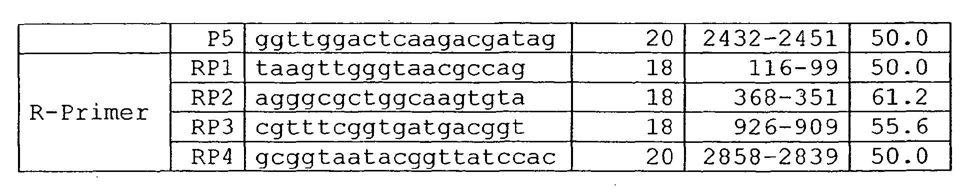

### (P1 to PR4: SEQ ID NO: 10 to SEQ ID NO: 18)

The positions of the primers shown in Table 1 are those renumbered designating the first base of the EcoRI cleavage site as position 1. As for the reverse primers RP1 to RP4 in Table 1, the positions are on the complementary chain.

Each primer having a base sequence shown in Table 1 was synthesized and purified by high-performance liquid chromatography (HPLC) and dissolved in a TE buffer solution to a final concentration of 10 pmol/µl to prepare a primer solution.

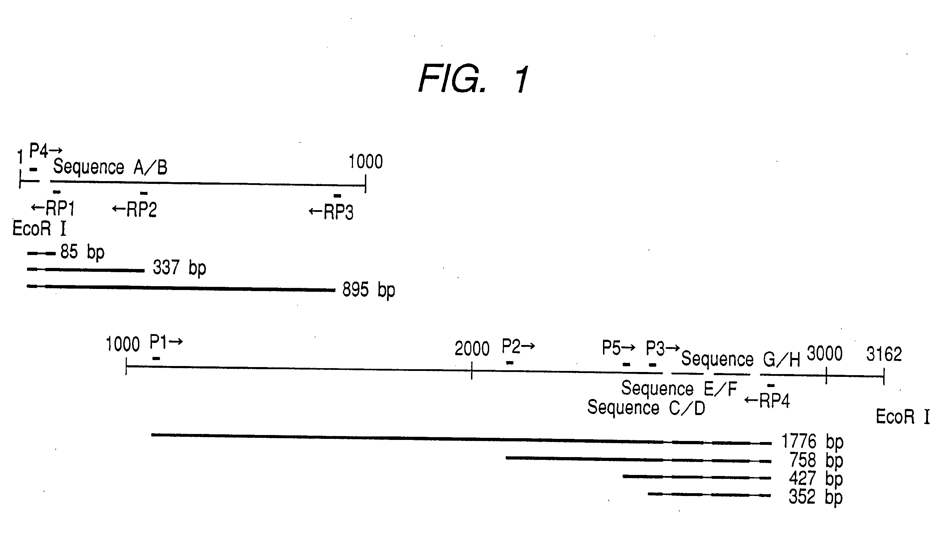

Fig. 1 shows the chain lengths of amplified products obtained by carrying out PCR amplification reactions using primers shown in Table 1 and the internal standard nucleic acid (EcoRI-digested pUC118 prepared in Example 1) as a template. Fig. 1 also shows combinations of forward primers and reverse primers used. Separately, 5'-fluorescent (rhodamine) labeled reverse primers were prepared in the same manner as with the above unlabeled primers to prepare fluorescence-labeled primer solutions.

### Example 3

### <Preparation of internal standard probes>

Internal standard probes shown below were prepared based on the information on the base sequence of the linear plasmid DNA pUC118 EcoRI digest prepared in Example 1.

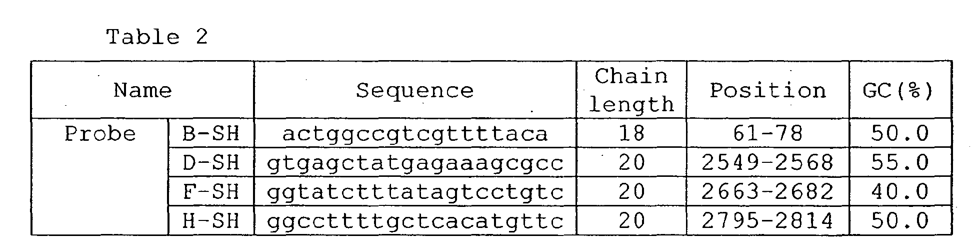

### (B-H: SEQ ID NO: 1 to SEQ ID NO: 4)

The positions of the probes shown in Table 2 are those renumbered designating the first base of the EcoRI cleavage site as position 1.

Four internal standard probes of which base sequences are shown in Table 2 were synthesized and then a sulfanyl group was introduced to the 5' terminal of each nucleic acid as a functional group to immobilize the probes in the DNA micro-array in accordance with a conventional method. After the introduction of the functional group, the internal standard probes were purified and freeze-dried. The freeze-dried internal standard probes were preserved in a freezer at -30°C.

Fig. 1 shows the positions of the target base sequence to which the four internal standard probes would hybridize. The position of each PCR product on the target base sequence is shown as well.

### Example 4

### <Preparation of external standard nucleic acids>

The following external standard nucleic acids were prepared based on information on the sequence of linear plasmid, pUC118 EcoR I Digest prepared in Example 1.

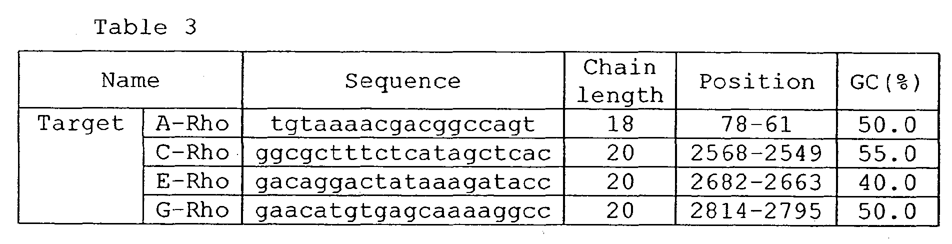

### (A-G: SEQ ID NO: 5 to SEQ ID NO: 8)

The positions of the probes shown in Table 3 are those renumbered designating the first base of the EcoRI cleavage site as position 1.

The four external standard nucleic acids are complementary to the four internal standard probes shown in Table 2.

A fluorescent dye (rhodamine) was introduced to the 5' terminals of the four external standard nucleic acids of which base sequences are shown in Table 3 by a convention method after synthesis, as a marker for detection and quantification. After the introduction of the fluorescent marker, the external standard nucleic acids were purified and dissolved in a TE buffer solution to a final concentration of 5 µM to prepare external standard nucleic acid solutions. The external standard nucleic acid solution were put in a light-shielded container and kept in a refrigerator at -30°C.

Fig. 1 shows the positions of the base sequences of the four external standard nucleic acids and the above four internal standard probes having complementary base sequences on the positions of the target base sequence.

### Example 5

### <Preparation of DNA micro-array>

### [1] Cleaning of glass substrate

A synthetic quartz glass substrate (size: 25 mm × 75 mm × 1 mm, manufactured by Iiyama Tokushu Glass Co., Ltd.) was placed in a heat-resistant and alkali-resistant rack and immersed in a cleaning liquid of a predetermined concentration for ultrasonic cleaning. After overnight immersion in the cleaning liquid, it was subjected to ultrasonic cleaning for 20 minutes. Subsequently, the substrate was taken out from the cleaning liquid and lightly rinsed with pure water, and ultrasonic cleaning was carried out in ultra-pure water for 20 minutes. The substrate was then immersed in a 1 N sodium hydroxide aqueous solution at 80°C for 10 minutes. After washing with pure water and with ultra-pure water, a cleaned quartz glass substrate for a DNA chip was obtained.

### [2] Surface treatment

A silane coupling agent (KBM-603, a product of Shin-Etsu Silicone Co., Ltd.) was dissolved in pure water to a concentration of 1 wt% and stirred at room temperature for 2 hours. Subsequently, the cleaned glass substrate was immersed in the aqueous solution of the silane coupling agent and kept at room temperature for 20 minutes. Then the glass substrate was pulled up, lightly washed with pure water on both surfaces, and dried by blowing nitrogen gas onto both sides. The dried substrate was then baked in an oven heated at 120°C for 1 hour to complete the coupling treatment, thereby amino groups were introduced onto the surface of the substrate.

Next, N-(6-maleimidocaproyloxy)succimido (EMCS)(a product of by Dojin Kagaku Kenkyujo Co., Ltd.) was dissolved in a solvent mixture of dimethyl sulfoxide and ethanol (volume ratio of 1:1) to a final concentration of 0.3 mg/ml to prepare an EMCS solution. The glass substrate baked and cooled was then immersed in the EMCS solution at room temperature for 2 hours. By this treatment, maleimido groups were introduced onto the substrate surface through the reaction between the succimido group of EMCS and the amino groups on the substrate surface introduced by the silane coupling agent treatment. Then the glass substrate was pulled up from the EMCS solution and washed with the above mixed solvent of dimethyl sulfoxide and ethanol. After further washing with ethanol, the surface-treated glass substrate was dried in a nitrogen gas atmosphere.

### [3] Probe DNA

The four internal standard probes prepared in Example 3 were dissolved in pure water to prepare solutions of various concentrations and compositions as shown in Table 4 below. Each internal standard probe solution was divided into aliquots and freeze-dried to remove water.

**Table 4**

| No. | Component | | Concentration |
|---|---|---|---|
| 1 | B-SH | One probe | 1.50 D/ml |
| 2 | D-SH | | |
| 3 | F-SH | | |
| 4 | H-SH | | |
| 5 | B-SH | One probe | 0.750 D/ml |
| 6 | D-SH | | |
| 7 | F-SH | | |
| 8 | H-SH | | |
| 9 | B-SH | One probe | 0.50 D/ml |
| 10 | D-SH | | |
| 11 | F-SH | | |
| 12 | H=SH | | |
| 13 | B=SH | One probe | 0.3750 D/ml each |
| 14 | D-SH | | |
| 15 | F-SH | | |
| 16 | H-SH | | |
| 17 | B+D | Mixture of two probes | 0.750 D/ml each |
| 18 | B+F | | |
| 19 | B+H | | |
| 20 | D+F | | |
| 21 | D+H | | |
| 22 | F+H | | |
| 23 | B+D+F | Mixture of three probes | 0.50 D/ml each |
| 24 | B+D+H | | |
| 25 | B+F+H | | |
| 26 | D+F+H | | |
| 27 | B+D+F+H | Mixture of four probes | 0.3750 D/ml each |

### [4] Discharge of DNA by BJ printer and bonding to substrate

An aqueous solution containing 7.5 wt% of glycerin, 7.5 wt% of thiodiglycol, 7.5 wt% of urea and 1.0 wt% of Acetylenol EH (produced by Kawaken Fine Chemical Co., Ltd.) was prepared. Then, an aliquot of each of 27 freeze-dried probe solutions shown in Table 4 was dissolved in a specific amount of the above mixed solvent to a specific concentration. The obtained DNA probe solutions were respectively charged into ink tanks for a bubble jet printer (trade name: BJF-850, manufactured by Canon Inc.), and the tank was mounted to a printing head.

The bubble jet printer used herein was modified to enable printing on a plane surface. This bubble jet printer can discharge ink droplets according to a pattern inputted using a certain file creation method, and can spot about 5 pico liter of the DNA solution at a pitch of about 120 µm.

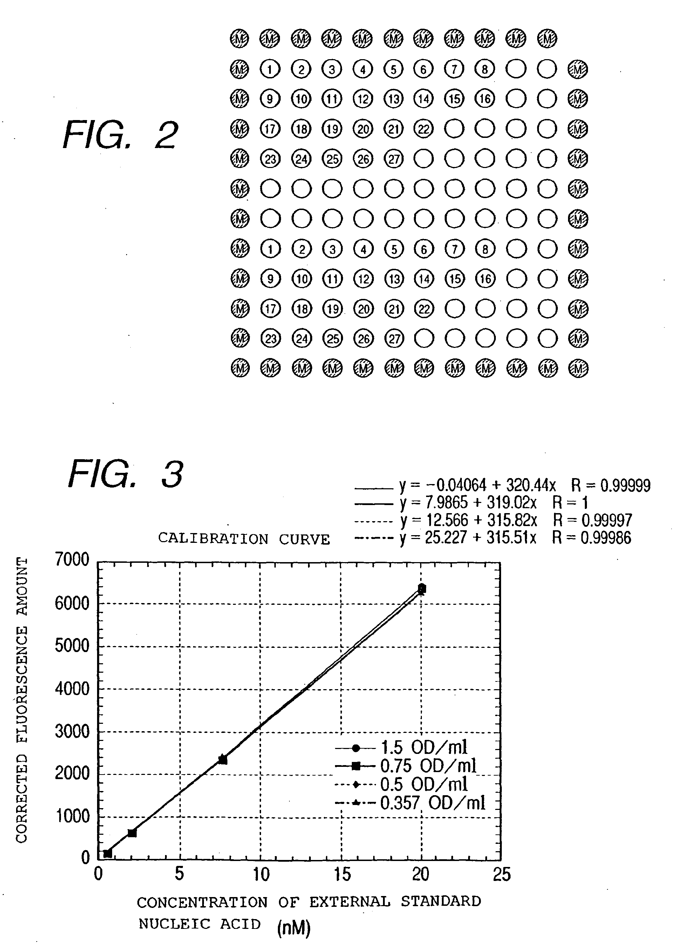

Subsequently, the 27 different probe solutions were spotted onto a surface-treated glass substrate, in accordance with the order shown in Fig. 2, using the above modified bubble jet printer. After confirming that intended spotting was carried out, the glass substrate was left to stand in a humidified chamber for 30 minutes to react the maleimido groups on the surface of the glass substrate with the sulfanyl groups at the 5' terminals of the DNA probes.

### [5] Washing

After 30 minutes of reaction, the DNA solutions remaining on the surface were washed away with a 10 mM phosphate buffer solution (pH of 7.0) containing 100 mM NaCl to obtain a gene chip (DNA probe array substrate) having immobilized single-stranded DNAs spotted in a matrix pattern on the surface of the glass substrate.

### Example 6

### <Calibration of substrate using external standard nucleic acid>

Eight DNA micro-arrays prepared in Example 5 were used. The eight DNA micro-arrays were immersed in a blocking solution having a composition shown below and left to stand at room temperature for 3 hours to carry out blocking treatment.

**Table 5**

| Composition of blocking solution | |
|---|---|
| Component | Content |
| Bovine serum albumin (produced by Sigma Co., Ltd.) | 2%(w/v) |
| NaCl | 100 mM |
| Phosphate buffer solution pH 7.0 | 10 mM |

Meanwhile eight different hybridization solutions containing external standard nucleic acid as shown in Table 6 were prepared. The basic solvent for these hybridization solutions was a 10 mM phosphate buffer solution (pH of 7.0) containing 100 mM NaCl (to be referred to as "basic buffer solution" hereinafter), and each of the external standard nucleic acids shown in Table 6 or a mixture thereof was dissolved in this basic buffer solution to a total nucleic acid concentration of 30 nM.

**Table 6**

| No. | External standard nucleic acid | Content |
|---|---|---|
| 1 | A | 30 nM each |
| 2 | G | 30 nM each |
| 3 | A+G | 15 nM each |
| 4 | C+G | 15 nM each |
| 5 | E+G | 15 nM each |
| 6 | A+C+G | 10 nM each |
| 7 | C+E+G | 10 nM each |
| 8 | A+C+E+G | 7.5 nM each |

In Table 6, abbreviations in the column of external standard nucleic acids designate the external standard nucleic acids shown in Table 3, indicating that corresponding external standard nucleic acids are contained.

DNA micro-arrays subjected to the blocking treatment and rinsed with the basic buffer solution were immersed in the eight hybridization solutions respectively and sealed up in a vinyl pack. A hybridization reaction was carried out in each DNA micro-array at 45°C for 3 hours.

After the completion of the reaction, each DNA micro-array was taken out from the vinyl pack and the following cleaning was carried out.

The DNA micro-array was washed with 2 × SSC + 0.1 % sodium dodecylsulfate (SDS) at 55°C for 5 minutes for three times and 0.1 x SCC at room temperature for 1 minute once. The composition of 2 × SSC was: NaCl 17.5 g/l, trisodium citrate · dihydrate 8.8 g/l, adjusted to pH 7.0 with sodium hydroxide.

After washing, extra liquid was blown off with air to dry the DNA micro-array. After drying, the fluorescence intensity at each spot was measured by using a DNA array scanner (GenePix 4000B, manufactured by Axon Co., Ltd.). The measurement data was analyzed by using analytical software (Genepix Pro 3.0) attached to the DNA array scanner.

### Example 7

### <Density calibration of immobilized probes>

The density ratio of the probes was calculated from the measured fluorescence intensities. The results are shown in Table 7.

**Table 7**

| No. | Content | | Concentration | Density ratio (%) |
|---|---|---|---|---|
| 1 | B-SH | One kind | 1.50 D/ml | 185 |
| 2 | D-SH | | | 189 |
| 3 | F-SH | | | 187 |
| 4 | H-SH | | | 189 |
| 5 | B-SH | One kind | 0.750 D/ml | 100 |
| 6 | D-SH | | | 100 |
| 7 | D-SH | | | 98 |
| 8 | H-SH | | | 101 |
| 9 | B-SH | One kind | 0.50 D/ml each | 67 |
| 10 | D-SH | | | 66 |
| 11 | F-SH | | | 66 |
| 12 | H-SH | | | 68 |
| 13 | B-SH | One kind | 0.3750 D/ml each | 51 |
| 14 | D-SH | | | 49 |
| 15 | F-SH | | | 51 |
| 16 | H-SH | | | 50 |

The density ratio was calculated as a relative ratio making the immobilization density of the DNA probe of Ink No. 5 to 100 %. The density ratios shown in Table 7 were determined by first determining the labeling rate of each external standard nucleic acid separately and then correcting the density ratio with the labeling rate.

In Table 7, the density ratios of Ink Nos. 1 to 4 (amounts of fluorescence) do not reach the theoretical value of 200 %, because the immobilized amount of the probe nucleic acid reached the permissible amount (saturation amount) of the substrate.

The density ratio of each probe in the mixed probe ink Nos. 17 to 27 shown in Table 4 was similar to the density ratio of the corresponding single probe of a corresponding concentration.

### Example 8

### <Creation of calibration curve using external standard nucleic acid>

The amount of each probe immobilized was measured from the results of Example 7 and a calibration curve was drawn using the correction data.

More specifically, hybridization experiments were conducted in the same manner as in Example 6 using the DNA micro-array prepared in Example 5. The external standard nucleic acid was added as a mixture in amounts shown below. Mixing ratio of the external standard nucleic acids:
- A-Rho:: 66.7 % (20 nM)
- C-Rho:: 25.0 % (7.5 nM)
- E-Rho:: 6.7 % (2.0 nM)
- G-Rho:: 1.7 % (0.5 nM)

The measurement and analysis were carried out in the same manner as in Example 6.

Calibration curves were drawn using the measurement results and the correction values of probe density obtained in Example 7. Fig. 3 shows the obtained calibration curves.

### Example 9

### <Creation of calibration curves using PCR products>

Calibration curves of PCR amplified products were drawn using the internal standard nucleic acid prepared in Example 1 and the internal standard primers prepared in Example 2.

First, a PCR amplification reaction was carried out using a combination of internal standard primers shown in Table 8 in accordance with a predetermined method. The composition of a PCR reaction solution is as follows. The reverse primers used in this example were primers whose 5' terminals were labeled with fluorescence prepared in Example 3.

| -PCR reaction solution- | |
|---|---|
| PCR premix reaction solution (2x) | 25 µl |
| pUC118 EcoR I Digest | 1 µl |
| Mixed primer (Table 8) | 6 µl |
| Pure water | 18 µl/50 µl in total |

Temperature cycle (24 cycles each including: 92°C for 10 sec; 62°C for 15 sec; and 72°C for 30 sec)

**Table 8**

| No. | Forward primer | Reverse primer | Primer concentration | Chain length of amplified product |
|---|---|---|---|---|
| 1 | P-4 | PR-2* | 5 nmol/ml each | 337 bp |
| 2 | | PR-3* | | 895 bp |
| 3 | P-1 | PR-4* | | 1776 bp |
| 4 | P-2 | | | 758 bp |
| | | | | |

| | | | | |
|---|---|---|---|---|
| * labeled with fluorescence. | | | | |

After the reaction, gel filtration was carried to purify the PCR amplified product. A TE buffer solution was used to adjust the volume of the PCR amplified product, and then the absorbance at 240 nm and the amount of fluorescence were measured to determine the amount of nucleic acid and the amount of the product labeled by fluorescence in order to obtain the labeling rate.

A hybridization reaction was carried out in the same manner as in Example 8 using the internal standard amplified product of known labeling rate to measure the amount of fluorescence derived from the internal standard amplified product bounding to the probe.

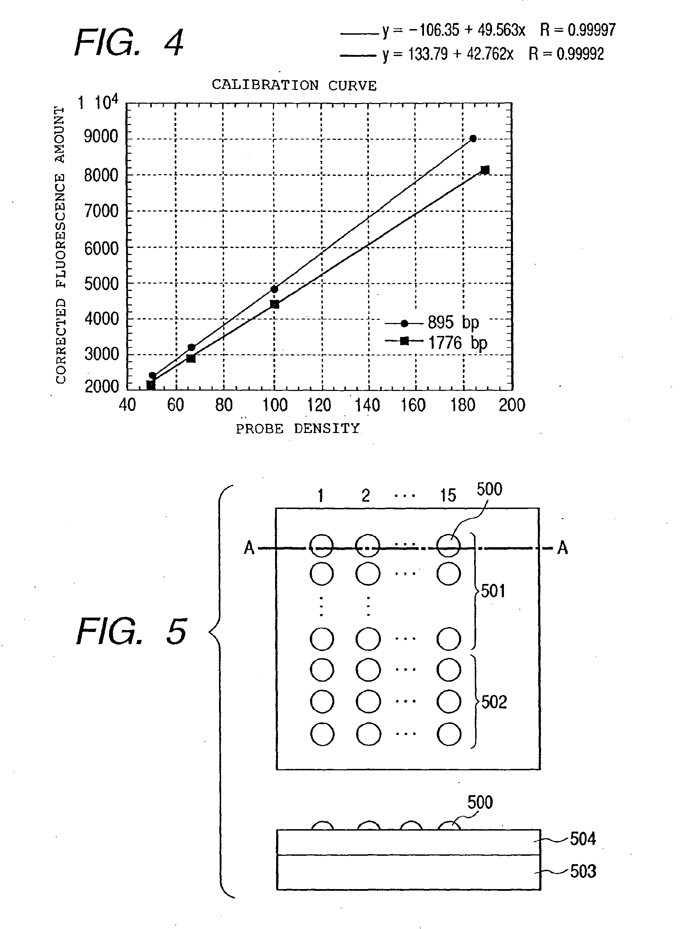

Calibration curves were drawn from the measurement results using the labeling rate of the internal standard amplified product and the correction value of probe density obtained in Example 7. Fig. 4 shows the obtained calibration curves.

When the calibration curves shown in Fig. 4 are reviewed, there is a difference in the slope between the calibration curves depending on the molecular weight (chain length) of the amplified products. That is, in order to determine the target nucleic acid with higher reliability, it is necessary to employ a combination of primers giving internal standard amplified products having the same chain length as the amplified product of the target nucleic acid.

The following examples show use of density standard probes formed by the same method as with the nucleic acid probes to determine the amount or density of the above nucleic acid probes.

### Example 10

Fig. 5 schematically shows the plane arrangement of an array of probe spots bonded to a substrate and the sectional structure of the probe array. Reference numeral 501 denotes a group of probes prepared under arbitrary conditions; and 502, a group of probes having a known nucleic acid concentration. In the sectional view, reference numeral 503 denotes a substrate; 504, a surface-treated layer made from an organic material; and 500, a nucleic acid probe. The process for producing this probe array shown in Fig. 5 using a known method (method 9 disclosed in JP H11-187900 A) will be described hereinbelow.

The cleaning [1] and surface treatment [2] of the glass substrate were carried out in the same manner as in Example 5.

### [3] Synthesis of probe DNA

Single-stranded nucleic acid having SEQ ID NO: 9 (40-mer of dT) was synthesized by a DNA manufacturer (Bex Co., Ltd.). A (SH) group was introduced into the 5' terminal of the single-stranded DNA having SEQ ID NO: 1 by using a thiol modifier (Glen Research Co., Ltd.). After the DNA synthesis, deprotection and the collection of DNA were carried out by conventional methods and HPLC was used for purification. The manufacturer did the steps from synthesis to purification.

SEQ ID NO: 9

5' HS-(CH2)6-O-PO2-O-TTTTTTTTTT TTTTTTTTTT TTTTTTTTTT

TTTTTTTTTT 3'

### [4] Discharge by thermal jet printer and bonding of DNA to substrate

A single-stranded DNA of SEQ ID NO: 1 described in [3] was dissolved in a solution containing 7.5 wt% of glycerin, 7.5 wt% of urea, 7.5 wt% of thioglycol and 1 wt% of acetylene alcohol (trade name: Acetylenol EH; produced by Kawaken Fine Chemical Co., Ltd.) to a final concentration of 8 µM.

The BC-50 printer head (manufactured by Canon Inc.) for the BJF-850 bubble jet printer (manufactured by Canon Inc.) using a bubble jet method (a kind of thermal jet method) was modified to discharge several hundreds µl of a solution. This modified head was mounted to a modified discharge-drawing machine so that ink could be discharged to a flat quartz substrate. The above DNA solution was injected in the modified tank of this head in an amount of several hundred µl and the EMCS-treated substrate was set in the drawing machine to spot the solution on the EMCS-treated surface. The discharge amount of the solution at one ejection was 4 pl/droplet and the spotted area was 20 mm × 30 mm on the substrate at a pitch of 200 dpi, that is, 127 µm intervals. Under the above conditions, the diameter of dots spotted in a 157 × 236 matrix pattern (157 rows, 236 columns) was about 50 µm.

After spotting, the substrate was left to stand in a humidifed chamber for 30 minuets for reaction of the maleimido group on the surface of the substrate and the sulfanyl group (-SH) at the 5' terminals of the nucleic acid probes in order to immobilize the DNA probes. Thereafter, the substrate was rinsed with pure water and preserved in pure water. Just before TOF-SIMS analysis, the DNA bonded substrate (DNA chip) was dried by blowing nitrogen gas and further dried in a vacuum desiccator. Under the above conditions, 15 DNA chips were produced in total.

Further, 25 DNA chips and 35 DNA chips were prepared by the same manner as above except that the concentration of the above single-stranded DNA (SEQ ID NO: 1) in the spotting solution was changed to 5 µM and 2.5 µM respectively. The above three types of DNA chips were designated as standard DNA chips.

Thereafter, a DNA chip was produced under the same conditions as above using a single-stranded DNA solution of an ordinary concentration (about 8 µM). In the above DNA chip, the first to third rows from the end thereof were formed respectively using the same three single-stranded DNA solutions as used in the preparation of the standard DNA chips. That is, probe spots were formed with the DNA solution having a concentration of 10 µM in the first row at the endmost position of the substrate, probe spots were formed with the DNA solution having a concentration of 5 µM in the second row, and probe spots were formed with the DNA solution having a concentration of 2.5 µM in the third row. The other rows (4th to 157th rows) were formed with a 8 µM solution of DNA of another lot. The number of probe arrays can be set to any value.

### Example 11

### <Determination of nucleic acid on nucleic acid chip>

The standard DNA chips produced in three different DNA concentrations were washed with an acid to dissolve DNA probes and the solution was concentrated to 1 ml or less under conditions that the acid solution did not scatter. Thereafter, ultra-pure water was added to the concentrated solution to a total volume of 1 ml. This solution was introduced into an ICP-MS apparatus to measure the mass of P (phosphorus). Based on this measurement value, in consideration of the number of dots used for the above measurement (number of spots), the average value of the number of DNA molecules per dot was determined for each DNA concentration. The number of substrates of each DNA concentration may be determined in consideration of the detection limit concentration of P of the ICP-MS apparatus (about 10 ppb) and the number of DNA molecules on the substrate, not limited to the number of substrates in Example 10.

### Example 12

### <TOF-SIMS measurement>

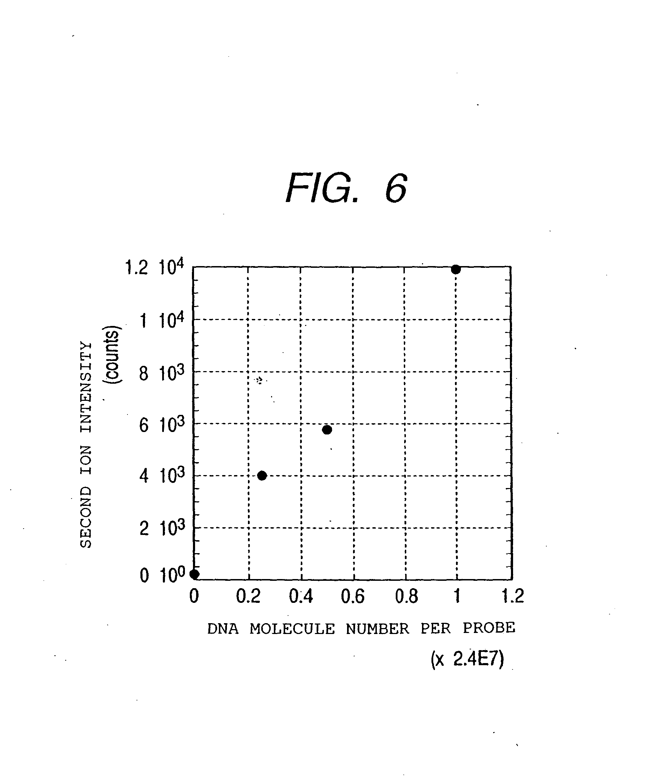

The DNA chip shown in Fig. 5 was conveyed to the analysis chamber of a time-of-flight type secondary ion mass spectrometer (TOFSIMS IV: manufactured by ION TOF Co., Ltd.) and irradiated with ions to the final primary ion dose density of 1 × 10¹² atoms/cm² under conditions shown in Table 1. The secondary ion PO₃⁻ (mass charge ratio of 78.958 amu (atom mass unit)) detected during this operation was integrated to obtain cumulative intensity. Fig. 6 shows a relationship between the average value of secondary ion intensity of the dots of one DNA concentration formed on one of the first to third rows and the DNA formation density (number of DNA molecules per dot). The intensity of PO₃⁻ measured with the surface-treated substrate right before the probe DNA immobilization was used as the background.

It was confirmed that the DNA formation density and the secondary ion intensity of the dots of each row were in proportion. When TOF-SIMS analysis was carried out under the same measurement conditions for a nucleic acid probe array prepared using a 8 µM nucleic acid solution and an ink jet printer to discharge the solution, the DNA formation density of each dot (number of DNA molecules per dot) obtained from a count value of PO₃⁻ was about 2.0 × 10⁷ and the variation in 10 dots selected at random was 20 % or less.

**Table 9**

| Measurement conditions of TOF-SIMS | | | |
|---|---|---|---|
| Primary ion | | Secondary ion | |
| Ion species | Ga⁺ | Ion species | PO₃⁻ |
| Acceleration voltage | 25 kV | Measurement area | 300µm × 300µm |
| Pulse | 2.5 kHz | Integrating circuit | 256 |

### Example 13

### <Imaging of formation density distribution>

The same nucleic acid probe array as in Example 12 was prepared, and its surface was scanned with primary ions to display the generated secondary ions corresponding to each scan point, the intensity of P⁻ obtained at each scanning point was classified into a plurality of intensity levels, and a dummy color was set for each level to quantitatively compare the intensity distribution of P⁻, that is, the distribution of surface density or formation density of nucleic acid (number of molecules of nucleic acid per probe dot).

As described above, according to the present invention, provided is a DNA micro-array which enables easy and detailed analysis. More specifically, detection of a nucleic acid which is the actual object and an assay work can be carried out concomitantly. Highly accurate assay results can be obtained because quantitative assay is carried out simultaneously with the detection operation. Therefore, the determination accuracy of the quantitative analysis of the molecules of a target nucleic acid is greatly improved by using the DNA micro-array according to the present invention. For quantitative analysis, internal standard amplification primers and external standard nucleic acids are used as a detection kit in accordance with the DNA micro-array according to the present invention so that a user can make use of the present invention very easily. Further, nucleic acid probe dots for use as density standards whose average nucleic acid density per dot has been determined and which are formed by the same method as the formation of the above nucleic acid probe dots are existent on the chip substrate, whereby the nucleic acid concentrations of nucleic acid probe dots having an unknown concentration arranged on the substrate can be determined by secondary ion mass spectrometry. Therefore, reproducibility and quantitavity are improved as compared with the prior art method.

Using the method of the present invention, the reliability of DNA micro-array products can be improved. It also enables to visualize the density distribution of nucleic acid probe dots formed in a matrix pattern in the DNA micro-array.

## Claims

1. A DNA micro-array for detecting nucleic acid molecules having target base sequences in a sample comprising a substrate and nucleic acid probes having base sequences substantially complementary to the target base sequences immobilized on the substrate, wherein the array contains additional probes of one or several kinds selected from the following probes:
probes for internal standard nucleic acids of one or several kinds which hybridize with said internal standards and are added for quantitative evaluation of PCR of said nucleic acid molecules having the target base sequences;
probes for external standard nucleic acids of one or several kinds which hybridize with said external standards and are added for evaluation of accuracy
of detection operation and for quantitative analysis of the amount of the probes having base sequences substantially complementary to the target base sequences;
and probes added for quantitative evaluation of the amount or density of said nucleic acid probes having base sequences substantially complementary to the target base sequences, which are immobilized by the same method as said nucleic acid probes.

2. A DNA micro-array for detecting nucleic acid molecules having target base sequences in a sample comprising a substrate and nucleic acid probes having base sequences substantially complementary to the target base sequences immobilized on the substrate, wherein the array contains additional probes of one or several kinds selected from the following probes:
probes for internal standard nucleic acids of one or several kinds which hybridize with said internal standards and are added for quantitative evaluation of PCR of said nucleic acid molecules having the target base sequences;
probes for external standard nucleic acids of one
or several kinds which hybridize with said external standards and are added for evaluation of accuracy of detection operation and for quantitative analysis of the amount of the probes having base sequences substantially complementary to the target base sequences;
in which said internal and/or external standard nucleic acids are added in order to quantitatively determine a concentration of the target nucleic acid molecules in the sample.

3. The DNA micro-array according to claim 2, wherein at least the internal standard probes or the external standard probes or both are immobilized on the substrate as a group of at least four levels of amount or density.

4. The DNA micro-array according to claim 2, wherein the internal standard probes include at least two probes corresponding to PCR products with different chain lengths derived from the internal standard nucleic acids.

5. The DNA micro-array according to claim 2, wherein the external standard probes include at least two probes each having a mutually different base sequence of any length each complementary to the external standard nucleic acids added.

6. The DNA micro-array according to claim 2, wherein the internal standard probes and the external standard probes are synthetic nucleic acids immobilized on the substrate.

7. The DNA micro-array according to claim 6, wherein the synthesized nucleic acid has a chain length of 15 to 75 bases.

8. A primer set for PCR of internal standard nucleic acids to be amplified together with target nucleic acids during an PCR of the nucleic acids having the target base sequences upon quantitatively detecting the nucleic acids having the target base sequences using a DNA micro-array, wherein
chain lengths of PCR products derived from the nucleic acids having the target base sequences are designed to be substantially equal to chain lengths of PCR products derived from the internal standard nucleic acids given by the primer set.

9. A kit for detecting a target base sequence, which contains a primer set for PCR of an internal standard nucleic acid to be amplified together with a target nucleic acid during PCR of the nucleic acid having the target base sequence upon quantitatively detecting the nucleic acid having the target base sequence using a DNA micro-array, comprising at least two of the primer sets according to claim 8 corresponding to different chain lengths when an amplified product derived from the nucleic acid having the target base sequence has at least two chain lengths.

10. The kit for detecting a target base sequence according to claim 9, wherein the primer sets include at least one primer set for an amplified product chain length of 200 bp or less, at least one primer set for an amplified product chain length of 200 to 500 bp, at least one primer set for an amplified product chain length of 500 to 2,000 bp and at least one primer set for an amplified product chain length of 2,000 bp or more.

11. A kit for detecting a target base sequence, which contains external standard nucleic acids to be added to a sample upon quantitatively detecting a target nucleic acid using a DNA micro-array, comprising at least two external standard nucleic acids which are synthesized nucleic acids labeled with a detectable marker.

12. The kit for detecting a target base sequence according to claim 11, wherein the marker comprises a fluorescent material, a radioactive material or a light emitting material.

13. A kit for detecting a target base sequence, which contains internal standard nucleic acids to be amplified together with a target nucleic acid during PCR of the nucleic acid having the target base sequence upon quantitatively detecting the nucleic acid having the target base sequence using a DNA micro-array, comprising at least two nucleic acids derived from microorganism or virus as internal standard nucleic acids having no homology with the target base sequence to be detected.

14. A DNA micro-array having the first set of nucleic acid probe dots including a plurality of target nucleic acids arranged in a matrix pattern on a substrate, further comprising the second set of nucleic acid probe dots for assay of amounts or a densities of the nucleic acids in said dots, which are formed by the same method as the formation of said first set of nucleic acid probe dots and arranged on part of a surface of the substrate having said second set of nucleic acid probe dots formed thereon and whose average nucleic acid density per dot is determined.

15. The DNA micro-array according to claim 14, further comprising a plurality of said second set of nucleic acid probe dots having different levels of average nucleic acid density with average nucleic acid density per dot determined as the nucleic acid probe dots for use as density standards.

16. The DNA micro-array according to claim 14, wherein the average nucleic acid density per dot of the nucleic acid probe dots having the determined average nucleic acid density per dot are determined by chemical analysis separately.

17. The DNA micro-array according to claim 16, wherein inductively coupled plasma mass spectrometry (to be abbreviated as ICP-MS hereinafter) is used for the chemical analysis for determining the average nucleic acid density per dot.

18. The DNA micro-array according to claim 14, wherein the nucleic acid probe comprises a single-stranded nucleic acid.

19. The DNA micro-array according to claim 14, wherein the nucleic acid probe including a single-stranded nucleic acid and a target nucleic acid introduced by hybridization of the nucleic acid probe are both existent on the substrate.

20. An analyzing method for a DNA micro-array having nucleic acid probe dots including a plurality of nucleic acids arranged in a matrix pattern on a substrate, **characterized in that**:
on part of a surface of the substrate having said second set of nucleic acid probe dots formed thereon, nucleic acid probe dots whose average nucleic acid density per each dot has been determined are formed as density standards by the same method as the formation of said first set of nucleic acid probe dots, where the density standard nucleic acid probe dots are a plurality of nucleic acid probe dots having different levels of average nucleic acid densities, whose average nucleic acid density per each dot has been determined; and
a nucleic acid concentration of the first set of nucleic acid probe in each dot having an undetermined concentration arranged on the substrate is determined by the secondary ion mass spectrometry by using a calibration curve drawn based on signal intensities of secondary ions detected when secondary ion mass spectrometry is carried out on the plurality of said second set of nucleic acid probe dots having the different levels of average nucleic acid densities,.

21. The analyzing method for a DNA micro-array according to claim 20, wherein time-of-flight type secondary ion mass spectrometry is used as the secondary ion mass spectrometry.

22. The analyzing method for a DNA micro-array according to claim 21, wherein a secondary ion intensity detected by the secondary ion mass spectrometry is an integral intensity (count value) of specific secondary ions derived from the nucleic acid probes and released from a fixed area applied with primary ions when a dose of the primary ions is set to a fixed value of 1 × 10¹⁴/cm² or less.

23. The analyzing method for a DNA micro-array according to claim 21, wherein a secondary ion intensity detected by the secondary ion mass spectrometry is an integral intensity (count value) of specific secondary ions derived from the nucleic acid probes and released from a fixed area applied with primary ions when the dose of the primary ions is set to a fixed value of 1 × 10¹²/cm² or less.

24. The analyzing method for a DNA micro-array according to any one of claims 21 to 23, wherein the secondary ions detected by the secondary ion mass spectrometry include an anion obtained by eliminating one hydrogen atom from a base of one of adenine, thymine, guanine, cytosine, and uracil, or an anion selected from the group consisting of P⁻, PO⁻ , PO₂⁻ and PO₃⁻ as the secondary ion derived from the nucleic acid probe.

25. The analyzing method for a DNA micro-array according to any one of claims 21 to 24, further comprising displaying a detection result as an image which shows secondary ion intensity two-dimensionally according to an application position of the primary ions, based on the secondary ion intensity detected by the secondary ion mass spectrometry.

26. A method of producing a DNA micro-array having nucleic acid probes arranged in a matrix pattern on a substrate, comprising, upon forming said second set of nucleic acid probe dots for use as density standards whose average nucleic acid density per each dot is determined on part of a surface of the substrate having said first nucleic acid probe dots formed thereon:
forming said first set of nucleic acid probe dots in the matrix pattern on the substrate; and
forming the second set of nucleic acid dots on the part of the surface of the substrate by the same method as the formation of said first set of nucleic acid probe dots , wherein the nucleic acid probe dots for use as the density standards whose average nucleic acid density per each dot is pre-determined.
